# FASCICULE DE BREVET EUROPEEN

(11) **EP 0 360 641 B1**
(45) Date de publication et mention de la délivrance du brevet: **30.11.1994**
(21) Numéro de dépôt: 89402329.0
(22) Date de dépôt: 23.08.1989
(51) Int. Cl.: C12N 15/70, C12N 15/67, C12N 15/62, C12P 21/02

(54) **Séquence d'ADN participant à la régulation de l'expression d'une séquence d'ADN codant pour un précurseur d'un polypeptide, vecteurs d'expression et procédé de production périplasmique du polypeptide**
DNA-Sequenz, beteiligt an der Regulation der Expression einer DNA-Sequenz, die einen Polypeptid-Precursor kodiert, Expressionsvektoren und Methode der periplasmatischen Herstellung des Polypeptides
DNA sequence participating in the regulation of the expression of a DNA sequence encoding a polypeptide precursor, expression vectors, and method for the periplasmic production of the polypeptide

(30) Priorité: 24.08.1988 FR 8811187
(43) Date de publication de la demande: 28.03.1990
(73) Titulaire: SANOFI, 75008 Paris (FR)
(72) Inventeur: Legoux, Richard, F-31460 Caraman (FR); Leplatois, Pascal, F-81470 Cuq Toulza (FR); Joseph-Liauzun, Evelyne, F-31650 Saint Orens De Gameville (FR)
(74) Mandataire: Gillard, Marie-Louise

(56) Documents cités:
- EP-A- 0 241 446
- EP-A- 0 245 217
- EP-A- 0 251 842
- FR-A- 2 597 114
- PHARMACIA MOLECULAR BIOLOGICALS CATALOGUE, Mai 1986, pages 96-99, Uppsala, SE; "Chemicals and equipment for molecular biology"
- JOURNAL OF MOLECULAR BIOLOGY, vol. 148, 1981, pages 107-127, Academic Press Inc. Ltd, Londres, GB; J. BROSIUS et al.: "Gene organization and primary structure of a ribosomal RNA operon from Escherichia coli"

## Description

L'invention concerne une séquence d'ADN participant à la régulation de l'expression d'une séquence d'ADN codant pour un précurseur d'un polypeptide. Elle concerne également les vecteurs d'expression dans lesquels une telle séquence est introduite. Elle concerne encore le procédé consistant à exprimer ces vecteurs dans une souche de bactéries gram négatives en vue d'une production périplasmique d'un polypeptide. Elle concerne plus particulièrement un procédé de production de l'hormone de croissance humaine.

On sait que les bactéries gram négatives et plus particulièrement les souches de l'espèce Escherichia coli sont des hôtes de choix pour la production de polypeptides hétérologues, c'est-à-dire de polypeptides normalement non synthétisés par la souche mise en oeuvre, et notamment de polypeptides d'origine eucaryote. Elles sont adaptées à l'obtention des polypeptides synthétisés sous la forme d'un pré-polypeptide appelé précurseur. Seule en effet la forme mature traverse la membrane cellulaire et vient s'accumuler dans le périplasme d'où on peut l'extraire par un simple choc osmotique. La purification du polypeptide ainsi obtenu est simplifiée car il est un composant majeur du périplasme.

Les avantages d'une telle production sont rendus d'autant plus appréciables que la transcription de la séquence d'ADN codant pour ledit précurseur, la traduction de l'ARN messager puis la traversée par le polypeptide mature de la membrane cellulaire sont performantes.

L'amélioration de la transcription a été largement étudiée. Elle concerne la conception de séquences promotrices de force élevée et de contrôle aisé. Les demandes de brevet EP-A-067540 et EP-A-018069 visent à protéger de telles séquences.

L'amélioration de la traduction a donné lieu à quelques travaux. C'est ainsi que dans la demande EP-A-0 241 446 on a décrit de nouvelles séquences d'ADN placées en amont de la séquence de Shine et Dalgarno dont la transcription conduit à une séquence sur l'ARN messager s'hybridant de manière spécifique avec les nucléotides 447 à 487 de l'ARN ribosomal 16S (ou ARNr 16S), constituant essentiel des ribosomes. La modification ainsi obtenue du site de fixation des ribosomes conduirait à rendre plus performante la traduction pour des protéines hétérologues synthétisées sous la forme d'une protéine mature N-méthionylée s'accumulant dans le cytoplasme bactérien.

La demanderesse a vérifié si l'une des constructions préférées décrites dans la demande EP-A-0 241 446 conviendrait à la production de polypeptides à localisation périplasmique. A défaut d'un résultat satisfaisant, elle a recherché l'introduction en amont de la séquence de Shine et Dalgarno d'autres séquences d'ADN dont la traduction conduirait à une séquence d'ARN messager capable de s'hybrider avec l'ARNr 16S. Il s'est avéré que de telles séquences sont nombreuses mais que peu d'entre elles ont l'efficacité attendue. Cette recherche a conduit par contre la demanderesse à identifier une séquence d'un grand intérêt.

L'invention concerne précisément selon un premier aspect une séquence d'ADN participant à la régulation de l'expression d'une séquence d'ADN codant pour un précurseur d'un polypeptide, caractérisée en ce qu'elle comporte n nucléotides, n étant compris entre 4 et 10 nucléotides, dont n-1 nucléotides au moins sont complémentaires des nucléotides 970 à 978 de l'ARNr 16S.

La séquence d'ARN ribosomal 16S concernée répond à la formule (a) :
3' AAGAAGCGC 5'
où
- A =: Adénosine monophosphate,
- C =: Cytidine monophosphate,
- G =: Guanosine monophosphate.

La séquence régulatrice selon l'invention répond de manière avantageuse à la formule (b) :
5' TTTTTCGCG 3'
où
- T =: Thymidine monophosphate,
- C =: Cytidine monophosphate,
- G =: Guanosine monophosphate.

La séquence (b) est complémentaire de la séquence (a) par 8 de ses 9 nucléotides, sa transcription détermine sur l'ARN messager correspondant une séquence 5′ UUUUUCGCG 3′ (U = Uracile) complémentaire pour 8 de ses 9 nucléotides de la séquence d'ARN ribosomal 16S comprise entre les nucléotides 970 et 980 5′ CGCGAAGAA 3. L'efficacité de cette séquence régulatrice (b) est d'autant plus inattendue que l'on sait que les nucléotides 972 à 975 CGAA de l'ARN ribosomal 16S sont normalement engagés dans une liaison avec les nucléotides 960 à 963 UUCG dudit ARN pour contribuer à la stabilisation de sa structure secondaire (H.F. NOLLER, Science, 212 (1981) 403-411).

L'invention ne se limite pas à cette séquence d'ADN particulière TTTTTCGCG. Elle concerne également toute séquence de type TTXTTCGCG où X = A, C ou G et toute séquence qui serait dérivée de cette dernière et en reprendrait une suite d'au moins 4 nucléotides à la condition que dans la séquence d'ARN messager correspondante 4 des nucléotides hybrident avec les nucléotides 970 à 978 de l'ARNr 16S.

La séquence selon l'invention est placée en amont de la séquence de Shine et Dalgarno stricto sensu. Elle ne doit pas en être éloignée de plus de 10 nucléotides. De préférence elle n'en est séparée que par 4 nucléotides.

Les autres séquences régulatrices placées de part et d'autre de la séquence selon l'invention peuvent revêtir les formes connues. En particulier, la nature du promoteur importe peu.

L'invention concerne selon un autre aspect les vecteurs d'expression dans lesquels a été insérée une séquence codant pour un précurseur d'un polypeptide et placée en aval et sous le contrôle d'une séquence régulatrice comportant une séquence selon l'invention.

Ces vecteurs d'expression peuvent être de toutes sortes. De préférence, il s'agit de plasmides.

L'invention concerne selon un autre aspect un procédé de production périplasmique d'un polypeptide. Ce procédé, qui consiste à transformer une souche de bactéries gram négatives par un vecteur d'expression portant une séquence codant pour un précurseur d'un polypeptide, et à cultiver les bactéries transformées de manière à permettre l'expression de cette séquence et la maturation du précurseur par franchissement de la membrane cytoplasmique par le polypeptide mature venant s'accumuler dans le périplasme, se caractérise par la mise en oeuvre d'un vecteur portant une séquence selon l'invention.

La nature de la souche bactérienne importe peu. Il est apparu en particulier que les souches portant une mutation affectant au moins l'un ou l'autre des gènes cya et crp telles que décrites dans EP-A-0245138 convenaient.

Le procédé selon l'invention est approprié à la production de polypeptides synthétisés sous la forme d'un précurseur. La séquence codant pour le précurseur peut correspondre soit à une séquence naturelle, soit à une séquence modifiée pour tout ou partie en ce qui concerne sa partie codant pour le peptide-signal. Il peut encore s'agir d'une séquence hybride associant une séquence codant pour un peptide-signal naturel ou non et une séquence codant pour le polypeptide mature.

Le procédé selon l'invention est particulièrement adapté à la production de polypeptides hétérologues par rapport à la souche mise en oeuvre. Il peut s'agir notamment de polypeptides d'origine eucaryote.

Selon un mode de réalisation, l'invention concerne précisément un procédé de production d'hormone de croissance humaine (ou hGH).

La présente invention va être maintenant décrite plus en détail à l'aide de l'exemple suivant pour lequel il sera fait référence aux trois figures annexées.

La figure 1 représente une carte de restriction du plasmide p163,1. Les différents segments de restriction sont marqués de manière arbitraire selon la légende ci-dessous :

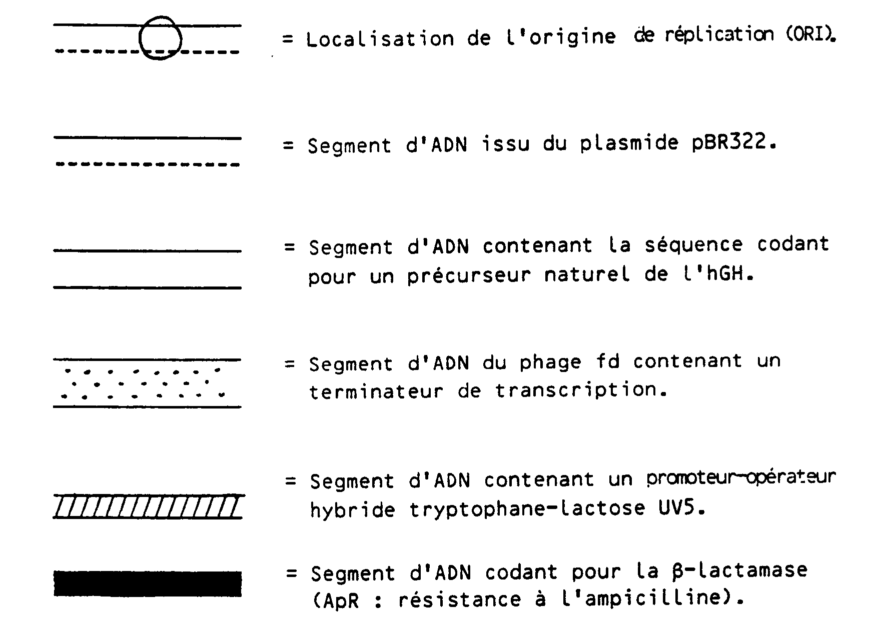
 = Localisation de l'origine de réplication (ORI).
 = Segment d'ADN issu du plasmide pBR322.
 = Segment d'ADN contenant la séquence codant
pour un précurseur naturel de l'hGH.
 = Segment d'ADN du phage fd contenant un
terminateur de transcription.
 = Segment d'ADN contenant un promoteur-opérateur
hybride tryptophane-lactose UV5.
 = Segment d'ADN codant pour la β-lactamase
(ApR : résistance à l'ampicilline).

La figure 2 représente la carte de restriction d'un plasmide dont les fragments PvuI-XhoI-BamHI(1) et PvuI-ORI-BamHI(2) proviennent respectivement des plasmides p163,1 et pBR327 et dont le petit fragment BamHI(2)-BamHI(1) est le fragment 3 décrit dans l'exemple 1 ci-après.

La figure 3 représente une carte de restriction commune aux plasmides p380,1 et p373,2. Les différents segments de restriction sont marqués de manière arbitraire selon la légende ci-dessous :

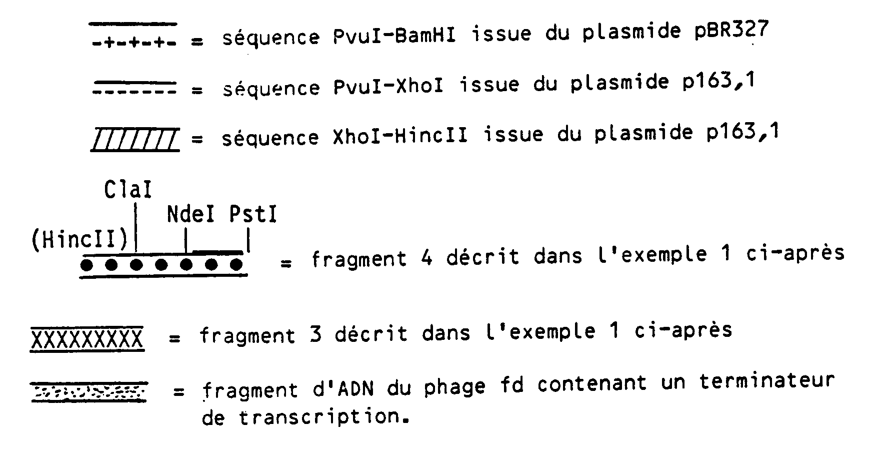
 = séquence PvuI-BamHI issue du plasmide pBR327
 = séquence PvuI-XhoI issue du plasmide p163,1
 = séquence XhoI-HincII issue du plasmide p163,1
ClaI
NdeI PstI
(HincII)
 = fragment 4 décrit dans l'exemple 1 ci-après
XXXXXXXXX = fragment 3 décrit dans l'exemple 1 ci-après
 = fragment d'ADN du phage fd contenant un terminateur
de transcription.

### EXEMPLE Production périplasmique d'hormone de croissance humaine

### 1. Bactéries et plasmides mis en oeuvre

Une souche bactérienne et quatre plasmides ont été mis en oeuvre.

Une souche de l'espèce Escherichia coli directement apparentée à la souche, décrite dans la demande de brevet EP-A-0245138 déposée à la Collection Nationale de Cultures de Microorganismes (CNCM, Paris, France) le 17 février 1986 sous la référence I-529, est mise en oeuvre. Cette souche porte une mutation cya par délétion et une mutation crp par délétion.

Les plasmides sont les plasmides dénommés ci-après p380,1, p381,1, p373,2 et p371,1.

### Construction des plasmides

La stratégie mise en oeuvre a fait appel à des fragments obtenus à partir de plasmides préexistants accessibles au public et à des fragments préparés par voie de synthèse selon les techniques maintenant couramment utilisées.

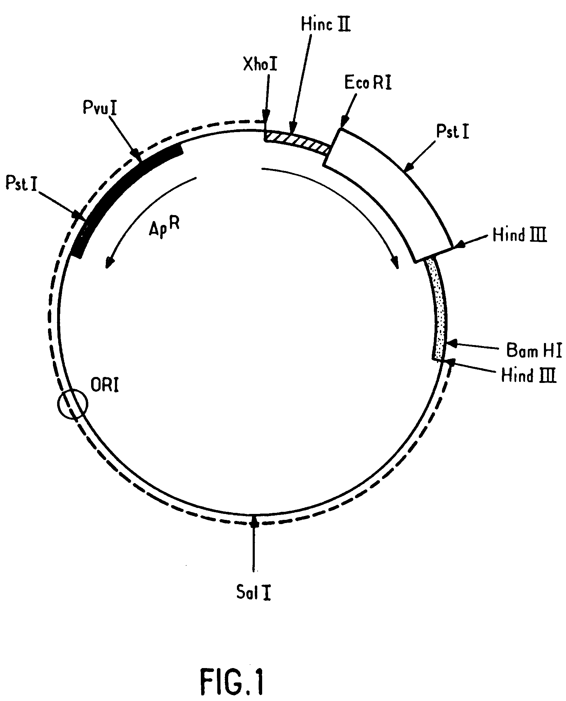

On a soumis le plasmide p163,1 (figure 1), décrit dans la demande de brevet EP-A-0245138 et déposé à la CNCM sous la référence I-530 le 17 février 1986, à une digestion par les enzymes PvuI et BamHI. Le fragment PvuI-BamHI - ci-après fragment 1 -, contenant le site d'action de l'enzyme de restriction XhoI, représenté sur la figure 1, a été purifié.

De même, on a soumis le plasmide pBR327, bien connu de l'homme de l'art, (cf. SOBERON, X et al., Gene, 9 (1980) 287-305) à une digestion par les enzymes PvuI et BamHI. Le fragment PvuI-BamHI - ci-après fragment 2 -, contenant l'origine de réplication, a été purifié.

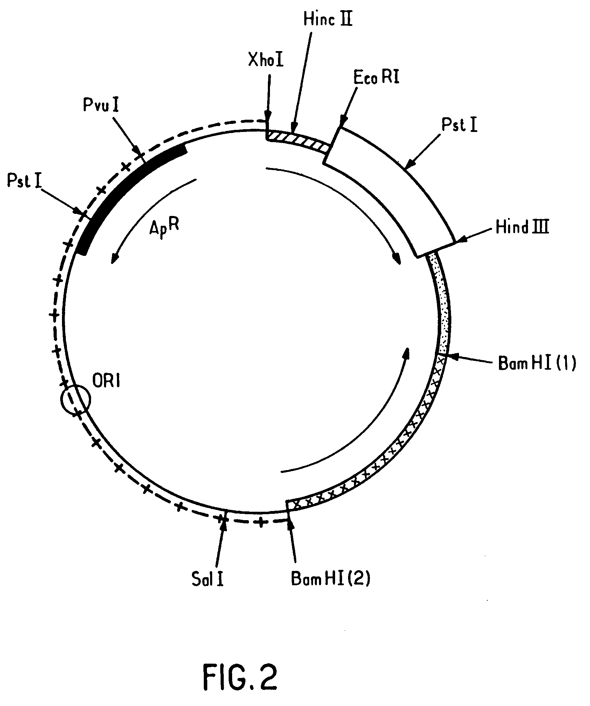

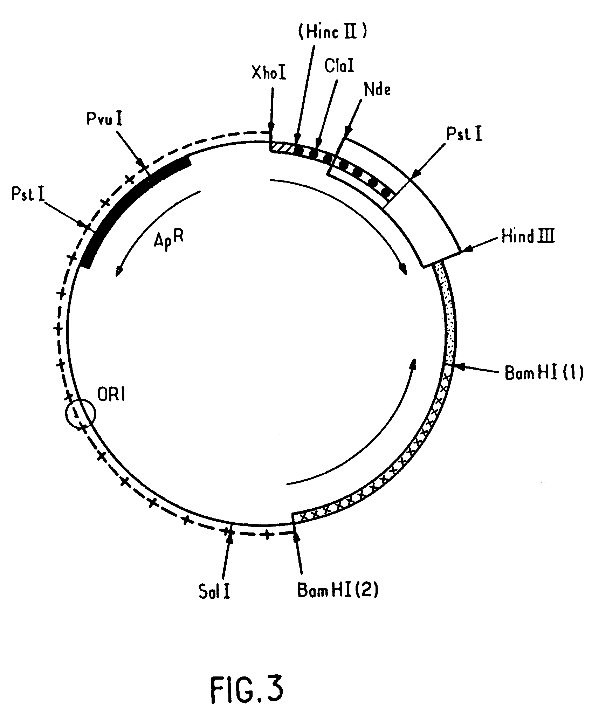

Puis on a préparé le fragment 3, qui est un fragment synthétique BamHI(1)-BamHI(2) contenant le gène lac i et son promoteur dont la séquence d'ADN est la suivante, sur laquelle les 2 extrémités du brin sont repérées par les nombres 1 et 2 pour préciser l'orientation du fragment dans les plasmides décrits aux figures 2 et 3 :

### FRAGMENT 3

BamHI(1)

5'    GATCC  GCGGAAGCAT  AAAGTGTAAA  GCCTGGGGTG  CCTAATGAGT
GAGCTAACTT  ACATTAATTG  CGTTGCGCTC  ACTGCCCGCT  TTCCAGTCGG
GAAACCTGTC  GTGCCAGCTG  CATTAATGAA  TCGGCCAACG  CGCGGGGAGA
GGCGGTTTGC  GTATTGGGCG  CCAGGGTGGT  TTTTCTTTTC  ACCAGTGAGA
CGGGCAACAG  CTGATTGCCC  TTCACCGCCT  GGCCCTGAGA  GAGTTGCAGC
AAGCGGTCCA  CGCTGGTTTG  CCCCACCACC  CGAAAATCCT  GTTTGATGGT
GGTTAACGGC  GGGATATAAC  ATGAGCTGTC  TTCGGTATCG  TCGTATCCCA
CTACCGAGAT  ATCCGCACCA  ACGCGCAGCC  CGGACTCGGT  AATGGCGCGC
ATTGCGCCCA  GCGCCATCTG  ATCGTTGGCA  ACCAGCATCG  CAGTGGGAAC
GATGCCCTCA  TTCAGCATTT  GCATGGTTTG  TTGAAAACCG  GACATGGCAC
TCCAGTCGCC  TTCCCGTTCC  GCTATCGGCT  GAATTTGATT  GCGAGTGAGA
TATTTATGCC  AGCCAGCCAG  ACGCAGACGC  GCCGAGACAG  AACTTAATGG
GCCCGCTAAC  AGCGCGATTT  GCTGGTGACC  CAATGCGACC  AGATGCTCCA
CGCCCAGTCG  CGTACCGTCT  TCATGGGAGA  AAATAATACT  GTTGATGGGT
GTCTGGTCAG  AGACATCAAG  AAATAACGCC  GGAACATTAG  TGCAGGCAGC
TTCCACAGCA  ATGGCATCCT  GGTCATCCAG  CGGATAGTTA  ATGATCAGCC
CACTGACGCG  TTGCGCGAGA  AGATTGTGCA  CCGCCGCTTT  ACAGGCTTCG
ACGCCGCTTC  GTTCTACCAT  CGACACCACC  ACGCTGGCAC  CCAGTTGATC
GGCGCGAGAT  TTAATCGCCG  CGACAATTTG  CGACGGCGCG  TGCAGGGCCA
GACTGGAGGT  GGCAACGCCA  ATCAGCAACG  ACTGTTTGCC  CGCCAGTTGT
TGTGCCACGC  GGTTGGGAAT  GTAATTCAGC  TCCGCCATCG  CCGCTTCCAC
TTTTTCCCGC  GTTTTCGCAG  AAACGTGGCT  GGCCTGGTTC  ACCACGCGGG
AAACGGTCTG  ATAACAGACA  CCGGCATACT  CTGCGACATC  GTATAACGTT
ACTGGTTTCA  CATTCACCAC  CCTGAATTGA  CTCTCTTCCG  GGCGCTATCA
TGCCATACCG  CGAAAGGTTT  TGCGCCATTC  GATGGTGTCC  G    3'
                                                BamHI(2)

Les fragments 1, 2 et 3 ont été alors ligués de manière à obtenir le plasmide intermédiaire décrit à la figure 2.

Ce plasmide a été soumis à une digestion partielle par les enzymes de restriction HincII et PstI. Le fragment HincII-PstI contenant l'origine de réplication et représenté sur la figure 3 a été ensuite ligué au fragment 4 représenté ci-après, qui est un fragment d'ADN synthétique portant une séquence codant pour les 44 premiers acides aminés d'un précurseur naturel de l'hGH et en amont de cette séquence des signaux de régulation.

### FRAGMENT 4 :

ClaI
5'    TCGAGCTGACTGACCTGTTGCTTATATTACATCGA
      AGCTCGACTGACTGGACAACGAATATAATGTAGCT
NdeI
TAGCGTATAATGTGTGGAATTGTGAGCGGATAACAATTTCACACAGXXXXXXXXXAAGAAGGAGATATACAT
ATCGCATATTACACACCTTAACACTCGCCTATTGTTAAAGTGTGTCXXXXXXXXXTTCTTCCTCTATATGTA

ATG GCT ACC GGA TCC CGG ACT AGT CTG CTC CTG GCT TTT GGC CTG CTC TGC CTG
TAC CGA TGG CCT AGG GCC TGA TCA GAC GAG GAC CGA AAA CCG GAC GAC ACG GAC
M A T G S R T S L L L A F G L L C L
-26

XbaI
CCC TGG CTT CAA GAG GGC AGT GCC TTC CCA ACC ATT CCC TTA TCT AGA CTT TTT
GGG ACC GAA GTT CTC CCG TCA CGG AAG GGT TGG TAA GGG AAT AGA TCT GAA AAA
P W L Q E G S A F P T I P L S R L F
-1 1

GAC AAC GCT ATG CTC CGC GCC CAT CGT CTG CAC CAG CTG GCC TTT GAC ACC TAC
CTG TTG CGA TAC GAG GCG CGG GTA GCA GAC GTG GTC GAC CGG AAA CTG TGG ATC
D N A M L R A H R L H Q L A F L T Y

PstI
CAG GAG TTT GAA GAA GCC TAT ATC CCA AAG GAA CAG AAG TAT TCA TTC CTG CA
GTC CTC AAA CTT CTT CGG ATA TAG GGT TTC CTT GTC TTC ATA AGT AAG G    5'
Q E F E E A Y I P K E Q K Y S F
44

Dans ce fragment, les acides aminés sont désignés par des lettres selon le code suivant :

| | |
|---|---|
| A = alanine | M = méthionine |
| C = cystéine | N = asparagine |
| D = acide aspartique | P = proline |
| E = acide glutamique | Q = glutamine |
| F = phénylalanine | R = arginine |
| G = glycine | S = sérine |
| H = histidine | T = thréonine |
| I = isoleucine | V = valine |
| K = lysine | W = tryptophane |
| L = leucine | Y = tyrosine |

Sont successivement soulignées les séquences -35 (TTGCTT) et -10 (TATAAT) de la séquence promotrice, et la séquence de Shine et Dalgarno bien connue de l'homme de l'art. Les X positionnés en amont de cette séquence représentent la suite particulière de 9 nucléotides : 
5' TTTAACTTT
---------
AAATTGAAA 5'
Le plasmide p380,1 a été ainsi obtenu.

A partir du plasmide p380,1 (figure 3), 3 autres plasmides ont été construits en y remplaçant le fragment ClaI - NdeI, matérialisé sur le fragment 4 ci-dessus, par chacun des 3 fragments ClaI - NdeI suivants :

### Fragment a :

ClaI

5' CGATAGCGTATAATGTGTGGAATTGTGAGCGGATAACA
TATCGCATATTACACACCTTAACACTCGCCTATTGT

NdeI

ATTTCACACAGTCTCCCCGCAAGAAGGAGATATACA
TAAAGTGTGTCAGAGGGGCGTTCTTCCTCTATATGTAT 5'

### Fragment b :

ClaI
5'  CGATAGCGTATAATGTGTGGAATTGTGAGCGGATAACA
      TATCGCATATTACACACCTTAACACTCGCCTATTGT

                                                    NdeI
ATTTCACACAGTCAATGAGCAAGAAGGAGATATACA
TAAAGTGTGTCAGTTACTCGTTCTTCCTCTATATGTAT   5'

### Fragment c :

ClaI
5' CGATAGCGTATAATGTGTGGAATTGTGAGCGGATAACA
TATCGCATATTACACACCTTAACACTCGCCTATTGT

NdeI
ATTTCACACAGTTTTTCGCGAAGAAGGAGATATACA
TAAAGTGTGTCAAAAAGCGCTTCTTCCTCTATATGTAT 5'

Les plasmides obtenus sont, avec le fragment a, le plasmide p381,1, avec le fragment b le plasmide p371,1 et avec le fragment c le plasmide p373,2

### 2. Méthodologie générale

Les essais réalisés ont consisté à cultiver les couples hôte-vecteur concernés, préalablement préparés (cf. § 2.1) dans des conditions telles qu'une biomasse suffisante soit obtenue (cf. § 2.2.), et que les cellules soumises à une induction produisent l'hGH (cf. § 2.3), à recueillir par choc osmotique les protéines contenues dans l'espace périplasmique (cf. § 2.4) et à doser l'hGH périplasmique (cf. § 2.5).

### 2.1 Préparation des couples hôte-vecteur

Les couples hôte-vecteur ont été préparés selon les techniques de transformation bactérienne connues de l'homme de l'art et qui sont décrites notamment dans les ouvrages ci-après.
- Molecular cloning - A Laboratory Manual -T. Maniatis, E.F. Fritsch and J. Sambrook - Cold Spring Harbor Laboratory - 1982.
- Experiments in Molecular Genetics - J.H. MILLER Cold Spring Harbor Laboratory - 1972.

### 2.2 Culture

### a) Ensemencement

Une colonie isolée obtenue sur milieu solide, (milieu LB + agar-agar), a été mise en suspension dans 5 ml d'un milieu (milieu LB).

Le milieu LB utilisé a les caractéristiques ci-après :
- ses composants introduits avant autoclavage sont :

| | |
|---|---|
| . de la Bactotryptone | 10 g |
| . de l'extrait de levure | 5 g |
| . du chlorure de sodium | 5 g |
| . de l'eau distillée | qsp 1 l |

- son pH est ajusté à 7,3 avant autoclavage ;
- de l'ampicilline est ajoutée après autoclavage à raison de 100 µg/ml.

### b) Incubation

La suspension préparée en a a été placée à 37°C pendant 18 h de façon que la culture arrive en phase de croissance stationnaire. La suspension dense obtenue a été diluée en milieu LB de manière à obtenir une valeur de densité optique lue à 600 nm - DO à 600 nm - proche de 0,03 puis 25 ml de cette suspension bactérienne ont été mis en incubation à 37°C sous agitation jusqu'à obtention d'une DO à 600 nm voisine de 0,3.

### 2.3 Induction

A la suspension bactérienne obtenue selon 2.2.b, on a ajouté de l'isopropyl-β-D-thiogalactose (ou IPTG) en quantité telle que sa concentration finale soit égale à 1mM ; l'IPTG a été ici utilisée pour provoquer le déclenchement et le maintien de la synthèse du précurseur de l'hGH en neutralisant l'action du répresseur venant normalement se fixer sur l'opérateur-Lactose.

La suspension additionnée d'IPTG a été maintenue sous agitation à 37°C pendant 2h30.

### 2.4 Choc osmotique

On s'est référé au protocole décrit par N.G. NOSSAL et L.A. HEPPEL dans "The Journal of Biological Chemistry 241 (1966) 3055-3063".

### a) Lavage au tris et à l'EDTA

Un échantillon de la suspension, telle qu'obtenue en 2.3. après induction, a été prélevé et centrifugé 5 minutes à 6 000 g.

Le culot a été repris dans un volume de tampon à pH 7 (solution A) (cf. ci-dessus) de façon que la suspension obtenue présente une DO à 600 nm voisine de 10.

Le tampon utilisé a été préparé par addition dans de l'eau distillée de :
. tri(hydroxyméthyl)aminométhane-HCl ou tris-HCl ajouté de façon à avoir une concentration finale de 30 mM.
. acide éthylènediaminetétraacétique ou EDTA ajouté de façon à avoir une concentration finale de 1 mM.

### b) Action du saccharose

La suspension obtenue en 2.4.a. a été centrifugée 5 minutes à 6 000 g.

Le culot a été repris délicatement à volume constant dans une solution B préparée extemporanément et correspondant à la solution A à laquelle est ajouté du saccharose à raison de 15 g pour 100 ml.

La suspension a été laissée 10 minutes à 20°C.

Puis elle a été centrifugée 5 minutes à 6 000 g.

Les tubes de centrifugation ont été placés dans de la glace fondante.

On a éliminé avec soin le surnageant et on lui a substitué (à volume constant) de l'eau désionisée et portée préalablement à la température de la glace fondante.

La suspension ainsi préparée (dont la DO à 600 nm était voisine de 10) a été laissée 5 minutes à 0°C.

### c) Recueil des protéines à localisation périplasmique

La suspension obtenue en 2.4.b. a été centrifugée 10 minutes à 18 000 g.

Le surnageant qui contenait les protéines à localisation périplasmique a été recueilli.

### 2.5 Dosage de l'hGH périplasmique

Le surnageant obtenu en 2.4.c a été soumis à une chromatographie de type liquide haute pression à l'aide d'un appareillage muni d'un système d'injection calibré et équipé d'un détecteur réglé sur 220 nm.

On a utilisé :
. une colonne phase inverse C 8 - 300 Angströms, en acier, de 10 cm de longueur et de 4,6 mm de diamètre interne (SYNCHROM référence C-8 R103-10),
. une phase mobile constituée par un gradient linéaire de 20 minutes passant respectivement de 70 volumes de solution S1 et 30 volumes de solution S2 à 40 volumes de solution S1 et 60 volumes de solution S2.

Les solutions S1 et S2 avaient les caractéristiques suivantes :
. S1 = eau purifiée contenant 0,1 % (v/v) d'acide trifluoroacétique,
. S2 = acétonitrile pour HPLC contenant 0,08 % (v/v) d'acide trifluoroacétique.
. Le débit était de 1 ml par minute.

La densité optique des fractions a été mesurée et la quantité d'hGH périplasmique exprimée en microgrammes par ml de surnageant a été déterminée par comparaison avec une gamme étalon préétablie·

### 2.6 Analyse par la technique dite du Western Blot

Il a été procédé successivement aux opérations suivantes:
- séparation électrophorétique sur gel (selon le protocole décrit par LAEMMLI, U.K. Nature 227 (1970) 680-685) des différentes protéines contenues dans chacun des surnageants obtenus selon 2.4.c. ; le gel utilisé était un gel de polyacrylamide (15 % p/v) contenant 0,5 % de dodécylsulfate de sodium.
- transfert desdites protéines contenues dans le gel sur un filtre de nitrocellulose (selon la technique de H. TOWBIN et al. Proc. Natl. Acad. Sci . USA 76 (1979) 4350-4354).
- Immunodétection, réalisée selon la technique de BURNETTE (W.W. BURNETTE Anal. Biochem. 112 ( 981) 195-203) ; elle implique successivement :
   . le rinçage du filtre de nitrocellulose pendant 10 min avec un tampon A (tris-HCl 10 mM, NaCl 170 mM, KI 1 mM),
   . la mise en contact du filtre de nitrocellulose pendant 30 min à 37°C avec un tampon B (tampon A additionné de sérum-albumine bovine à raison de 3 g pour 100 ml),
   . la mise en contact du filtre de nitrocellulose pendant 18 h à 20°C avec un immunsérum (un anticorps polyclonal reconnaissant l'hGH mature et son précurseur),
   . le rinçage du filtre de nitrocellulose avec le tampon B,
   . la mise en contact du filtre de nitrocellulose pendant 6 h à 20°C avec une solution de protéine A marquée à l'iode 125 à 0,1 microcurie par ml,
   . le rinçage du filtre avec le tampon A,
   . le séchage du filtre entre deux feuilles absorbantes,
   . la mise au contact d'un film radiographique,
   . la révélation du film.

### 3. Résultats

### - Présentation

Il a été noté que, dans les conditions opératoires retenues, l'hGH périplasmique était présente pour environ 98 % des molécules sous sa forme mature quels que soient la souche bactérienne et le plasmide utilisés.

Les résultats du dosage sont reportés dans le tableau ci-après :

| | | Plasmide testé | | | |
|---|---|---|---|---|---|
| | | 371,1 | 373,2 | 380,1 | 381,1 |
| 1 | DO à 600 nm mesurée sur la suspension obtenue en 2.3 après induction | 1,38 | 1,32 | 0,30 | 1,38 |
| 2 | hGH périplasmique mesurée en microgrammes par ml de surnageant recueilli après choc osmotique et ramené à une turbidité telle que DO à à 600 nm = 1 | 0,73 | 2,13 | 3,56 | 0,24 |

### - Interprétation

a) La ligne 1 rend compte du taux de croissance des cellules bactériennes. Il apparaît clairement que le plasmide 380,1 interdit une croissance cellulaire convenable.
b) La ligne 2 rend compte de la capacité de chaque cellule à produire de l'hGH périplasmique. On ne peut tenir compte du résultat obtenu avec le plasmide 380,1 compte tenu de la conclusion notée ci-dessus. Il est clair que, des 3 autres plasmides, c'est le plasmide 373,2 qui offre les meilleures performances en permettant une production près de 10 fois plus élevée, 3 fois plus élevée que ne le permettent respectivement les plasmides p371,1 et p381,1.

## Revendications (Revendications pour l'(les) Etat(s) contractant(s) suivant(s): AT, BE, CH, LI, DE, FR, GB, IT, LU, NL, SE)

1. Séquence d'ADN participant à la régulation de l'expression d'une séqeunce d'ADN codant pour un précurseur d'un polypeptide, caractérisée en ce qu'elle répond à la formule :
5' TTXTTCGCG 3'
dans laquelle X = A, C, G ou T
dans laquelle :
A = Adénosine monophosphate
C = Cytidine monophosphate
G = Guanosine monophosphate
T = Thymidine monophosphate

2. Vecteur d'expression portant une séquence d'ADN codant pour un précurseur d'un polypeptide hétérologue, caractérisé en ce qu'il porte une séquence régulatrice selon la revendication 1.

3. Vecteur selon la revendication 2, caractérisé en ce que la séquence d'ADN selon la revendication 1 est placée en amont de la séquence de Shine et Dalgamo, ladite séquence d'ADN n'étant pas éloignée de plus de 10 nucléotides de la séquence de Shine et Dalgarno AGGA.

4. Vecteur selon l'une des revendications 2 ou 3, caractérisé en ce qu'il est un plasmide.

5. Procédé de production périplasmique d'un polypeptide consistant à transformer une souche de bactéries gram négatives par un vecteur d'expression portant une séquence codant pour un précurseur d'un polypeptide, à cultiver les bactéries transformées de manière à permettre l'expression de la séquence codant pour le précurseur et la maturation du précurseur par franchissement de la membrane cytoplasmique par le polypeptide mature venant s'accumuler dans le périplasme, caractérisé en ce que ledit vecteur est un vecteur selon l'une des revendications 2 ou 3.

6. Procédé de production périplasmique d'hormone de croissance humaine selon la revendication 5.

7. Procédé selon l'une des revendications 5 ou 6, caractérisé en ce que la souche de bactéries gram négatives est une souche E.coli.

8. Procédé selon la revendication 7, caractérisé en ce que la souche E. coli porte une mutation cya par délétion et une mutation crp par délétion.

## Revendications (Revendications pour l'(les) Etat(s) contractant(s) suivant(s): ES)

1. Procédé de production périplasmique d'un polypeptide consistant à transformer une souche de bactéries gram négatives par un vecteur d'expression portant une séquence codant pour un précurseur d'un polypeptide, à cultiver les bactéries transformées de manière à permettre l'expression de la séquence codant pour le précurseur et la maturation du précurseur par franchissement de la membrane cytoplasmique par le polypeptide mature venant s'accumuler dans le périplasme, caractérisé en ce que ledit vecteur porte une séquence régulatrice de l'expression de la séquence d'ADN codant pour le précurseur du polypeptide, répondant à la formule :
5' TTXTTCGCG 3'
dans laquelle X = A, C, G ou T
dans laquelle :
A = Adénosine monophosphate
C = Cytidine monophosphate
G = Guanosine monophosphate
T = Thymidine monophosphate

2. Procédé selon la revendication 1, caractérisé en ce que la séquence d'ADN régulatrice est placée en amont de la séquence de Shine et Dalgarno, ladite séquence d'ADN n'étant pas éloignée de plus de 10 nucléotides de la séquence de Shine et Dalgamo AGGA.

3. Procédé selon la revendication 1 pour la production périplasmique d'hormone de croissance humaine.

4. Procédé selon l'une quelconque des revendications 1 à 3, caractérisé en ce que la souche de bactéries gram négatives est une souche E.coli.

5. Procédé selon la revendication 4, caractérisé en ce que la souche E.coli porte une mutation cya par délétion et une mutation crp par délétion.

## Claims (Claims for the following Contracting State(s): AT, BE, CH, LI, DE, FR, GB, GR, IT, LU, NL, SE)

1. DNA sequence participating in the regulation of the expression of a DNA sequence coding for a precursor of a polypeptide, characterized in that it has the formula:
5' TTXTTCGCG 3'
in which X = A, C, G or T
in which:
A = Adenosine monophosphate
C = Cytidine monophosphate
G = Guanosine monophosphate
T = Thymidine monophosphate

2. Expression vector carrying a DNA sequence coding for a precursor of a heterologous polypeptide, characterized in that it carries a regulatory sequence according to claim 1.

3. Vector according to claim 2, characterized in that the DNA sequence according to claim 1 is located upstream from the Shine-Dalgarno sequence, said DNA sequence not being more than 10 nucleotides away from the AGGA Shine-Dalgarno sequence.

4. Vector according to one of claims 2 or 3, characterized in that it is a plasmid.

5. Process for the periplasmic production of a polypeptide, which consists in transforming a strain of Gram-negative bacteria with an expression vector carrying a sequence coding for a precursor of a polypeptide, and in cultivating the transformed bacteria so as to permit the expression of the sequence coding for the precursor and the maturation of the precursor by passage through the cytoplasmic membrane of the mature polypeptide accumulating in the periplasm, characterized in that said vector is a vector according to one of claims 2 or 3.

6. Process for the periplasmic production of human growth hormone according to claim 5.

7. Process according to one of claims 5 or 6, characterized in that the strain of Gram-negative bacteria is an E. coli strain.

8. Process according to claim 7, characterized in that the E. coli strain carries a cya mutation by deletion and a crp mutation by deletion.

## Claims (Claims for the following Contracting State(s): ES)

1. Process for the periplasmic production of a polypeptide, which consists in transforming a strain of Gram-negative bacteria with an expression vector carrying a sequence coding for a precursor of a polypeptide, and in cultivating the transformed bacteria so as to permit the expression of the sequence coding for the precursor and the maturation of the precursor by passage through the cytoplasmic membrane of the mature polypeptide accumulating in the periplasm, characterized in that said vector carries a sequence that regulates the expression of the DNA sequence coding for the precursor of the polypeptide, having the formula:
5' TTXTTCGCG 3'
in which X = A, C, G or T
in which:
A = Adenosine monophosphate
C = Cytidine monophosphate
G = Guanosine monophosphate
T = Thymidine monophosphate

2. Process according to claim 1, characterized in that the regulatory DNA sequence is located upstream from the Shine-Dalgarno sequence, said DNA sequence not being more than 10 nucleotides away from the AGGA Shine-Dalgarno sequence.

3. Process according to claim 1 for the periplasmic production of human growth hormone.

4. Process according to any one of claims 1 to 3, characterized in that the strain of Gram-negative bacteria is an E. coli strain.

5. Process according to claim 4, characterized in that the E. coli strain carries a cya mutation by deletion and a crp mutation by deletion.

## Patentansprüche (Patentansprüche für folgende(n) Vertragsstaat(en): AT, BE, CH, LI, DE, FR, GB, GR, IT, LU, NL, SE)

1. DNA-Sequenz, die an der Regulation der Expression einer für einen Polypeptid-Prekursor kodierenden DNA-Sequenz beteiligt ist, dadurch gekennzeichnet, daß sie der Formel:
5' TTXTTCGCG 3'
entspricht, worin X = A, C, G oder T,
worin:
A = Adenosinmonophosphat
C = Cytidinmonophosphat
G = Guanosinmonophosphat
T = Thymidinmonophosphat

2. Expressionsvektor, der eine für einen Prekursor eines heterologen Polypeptids kodierende DNA-Sequenz trägt, dadurch gekennzeichnet, daß er eine regulatorische Sequenz nach Anspruch 1 aufweist.

3. Vektor nach Anspruch 2, dadurch gekennzeichnet, daß die DNA-Sequenz gemäß Anspruch 1 oberhalb der Shine-Dalgarno-Sequenz plaziert ist, wobei die DNA-Sequenz um nicht mehr als 10 Nukleotide von der Shine-Dalgarno-Sequenz AGGA entfernt ist.

4. Vektor nach einem der Ansprüche 2 oder 3, dadurch gekennzeichnet, daß er ein Plasmid ist.

5. Verfahren zur periplasmatischen Produktion eines Polypeptids, welches darin besteht, daß ein Stamm von gramnegativen Bakterien durch einen eine für einen Polypeptid-Prekursor kodierende Sequenz aufweisenden Expressionsvektor transformiert wird, die transformierten Bakterien derart kultiviert werden, daß die Expression der für den Prekursor kodierenden Sequenz und die Reifung des Prekursors durch Brechen der zytoplasmatischen Membran durch das sich im Periplasma angesammelte reife Polypeptid möglich sind, dadurch gekennzeichnet, daß der Vektor ein Vektor nach einem der Ansprüche 2 oder 3 ist.

6. Verfahren zur periplasmatischen Produktion von menschlichem Wachstumshormon nach Anspruch 5.

7. Verfahren nach einem der Ansprüche 5 oder 6, dadurch gekennzeichnet, daß der gramnegative Bakterienstamm ein E. coli-Stamm ist.

8. Verfahren nach Anspruch 7, dadurch gekennzeichnet, daß der E. coli-Stamm eine cya-Mutation durch Deletion und eine crp-Mutation durch Deletion aufweist.

## Patentansprüche (Patentansprüche für folgende(n) Vertragsstaat(en): ES)

1. Verfahren zur periplasmatischen Produktion eines Polypeptids, welches darin besteht, daß ein Stamm von gramnegativen Bakterien durch einen eine für einen Polypeptid-Prekursor kodierende Sequenz aufweisenden Expressionsvektor transformiert wird, die transformierten Bakterien derart kultiviert werden, daß die Expression der für den Prekursor kodierenden Sequenz und die Reifung des Prekursors durch Brechen der zytoplasmatischen Membran durch das sich im Periplasma angesammelte reife Polypeptid möglich sind, dadurch gekennzeichnet, daß der Vektor eine regulatorische Sequenz der Expression der für den Polypeptid-Prekursor kodierenden DNA-Sequenz der Formel:
5' TTXTTCGCG 3'
worin X = A, C, G oder T,
worin:
A = Adenosinmonophosphat
C = Cytidinmonophosphat
G = Guanosinmonophosphat
T = Thymidinmonophosphat,
aufweist.

2. Verfahren nach Anspruch 1, dadurchgekennzeichnet` daß die DNA-Sequenz gemäß Anspruch 1 oberhalb der Shine-Dalgarno-Sequenz plaziert wird, wobei die DNA-Sequenz um nicht mehr als 10 Nukleotide von der Shine-Dalgarno-Sequenz AGGA entfernt ist.

3. Verfahren nach Anspruch 1 zur periplasmatischen Produktion von menschlichem Wachstumshormon.

4. Verfahren nach einem der Ansprüche 1 bis 3, dadurch gekennzeichnet, daß der gramnegative Bakterienstamm ein E. coli-Stamm ist.

5. Verfahren nach Anspruch 4, dadurch gekennzeichnet, daß der E. coli-Stamm eine cya-Mutation durch Deletion und eine crp-Mutation durch Deletion aufweist.
